# EUROPÄISCHE PATENTSCHRIFT

(11) **EP 0 529 202 B1**
(45) Veröffentlichungstag und Bekanntmachung des Hinweises auf die Patenterteilung: **12.04.1995**
(21) Anmeldenummer: 92108114.7
(22) Anmeldetag: 14.05.1992
(51) Int. Cl.: G02C 5/12, G02C 5/16, G02C 5/14

(54) **Brille mit Polsterteil an den Bügelenden bzw. am Nasenauflageteil**
Spectacles with cushion part at the temple ends and respectively at the nose pad support
Lunettes avec partie coussin à l'embout des branches, respectivement au support de plaquettes de nez

(30) Priorität: 30.08.1991 DE 9110752 U; 15.02.1992 DE 9201984 U
(43) Veröffentlichungstag der Anmeldung: 03.03.1993
(73) Patentinhaber: UVEX WINTER OPTIK GmbH, D-90766 Fürth (DE)
(72) Erfinder: Wiedner, Klaus, W-8510 Fürth/Bay. (DE)
(74) Vertreter: Rau, Manfred, Dr. Dipl.-Ing.

(56) Entgegenhaltungen:
- EP-A- 0 042 712
- WO-A-91/06885
- CH-A- 656 234
- DE-A- 3 714 032
- DE-U- 8 703 262
- DE-U- 9 110 752
- DE-U- 9 201 984
- FR-A- 2 326 717
- FR-A- 2 339 877
- US-A- 3 953 114

## Beschreibung

Die Erfindung richtet sich auf eine Brille umfassend einen Rahmen und Bügel aus einem relativ harten Kunststoff oder Metall mit wenigstens einem Polsterteil aus einem relativ weichen Kunststoff an den Bügelenden und/oder an den Nasenauflageteilen des Rahmens.

Brillenrahmen aus einem relativ harten Kunststoff oder aus Metall weisen zwar einerseits eine hohe Stabilität auf und ermöglichen dementsprechend auch elegante und leichte Konstruktionen, andererseits kann es vor allem im Bereich des Nasenrückens und hinter den Ohren zu Druckstellen kommen, insbesondere wenn der Träger den Kopf häufig oder mit stärkeren Richtungswechseln bewegt, wie dies gerade dann der Fall ist, wenn solche Brillenrahmen bei Arbeitsschutzbrillen oder bei Sportbrillen eingesetzt werden.

Um hier Abhilfe zu schaffen, ist es bekannt, Polsterteile aus weicherem Kunststoff bzw. Polsterkissen aus weicherem Kunststoff mit Flüssigkeits- oder Lufteinschluß zu verwenden. Derartige Polsterkissen sind beispielsweise aus der gattungsgemäßen CH 656 234 A5 im Zusammenhang mit einer Arbeitsschutzbrille bekannt.

Die meisten derartigen kissenartigen Polsterteile werden an der Brille durch Kleben oder durch Einrasten in Ausnehmungen befestigt, d.h. es handelt sich um gesondert hergestellte Teile, welche anschließend montiert werden müssen und sich naturgemäß auch wieder unbeabsichtigt vom Brillenrahmen lösen können.

Andere vorbekannte Konzeptionen sehen vor, z.B. das gesamte Bügelende aus einem weicheren Kunststoff zu spritzen, um Druckstellen zu vermeiden. Dies führt andererseits wieder dazu, daß die Festigkeit des Sitzes leidet, was bei Arbeitsschutz- und Sportbrillen wiederum besonders nachteilig ist.

Letztendlich ist es auch bekannt, zur Erzielung einer Polsterwirkung an den relevanten Stellen im Bereich der Nasenauflage und der Bügelenden einen weicheren Kunststoff aufzuspritzen. Hierdurch wird zwar die Druckgefahr etwas reduziert, aber eine wirklich gute Abpolsterung nicht erreicht.

Hiervon ausgehend liegt der Erfindung die Aufgabe zugrunde, eine Brille der eingangs genannten Art mit Polsterteilen derart auszugestalten, daß eine dem jeweiligen Anwendungszweck individuell anpaßbare Weichheit der Polsterung auf herstellungstechnisch einfache Weise realisierbar ist.

Diese Aufgabe wird erfindungsgemäß dadurch gelöst, daß das Polsterteil als aus Kunststoff angespritzte, relativ dünnwandige Aufwölbung ausgebildet ist.

Eine derartige Aufwölbung weist sehr gute Federeigenschaften auf, die zudem durch die entsprechende Auswahl der Härte des Kunststoffes, der Geometrie der Aufwölbung und auch der Schichtdicke im Bereich der Aufwölbung sehr gut einstellbar sind.

In weiterer Ausgestaltung der Erfindung ist vorgesehen, daß an einem tragenden Grundkörper eines Nasenauflageteils bzw. des Ohrabschnitts des Bügels aus relativ hartem Kunststoff ein Polsterteil aus relativ weichem Kunststoff einstückig angespritzt ist.

Dementsprechend wird also die Federwirkung nicht nur durch die dünne, blasenartige Konfiguration des Polsterteils bestimmt, sondern auch durch den verwendeten, gegenüber dem tragenden Grundmaterial weicheren Kunststoff.

Vorteilhafterweise umschließt das blasenartig aufgewölbte Polsterteil einen etwa ovalen Grundflächenbereich, der in dem der Auflageseite abgewandten Bereich offen ist.

Es ist also erfindungsgemäß insoweit keine geschlossene Luftblase vorgesehen, sondern eine einseitig offene Blase.

Das Polsterteil kann günstigerweise einen verstärkten freien Außenrand aufweisen, durch dessen Dimensionierung die Festigkeit des Teils einstellbar ist.

Bei Realisierung der Erfindung im Nasenbereich kann vorgesehen sein, daß das Nasenauflageteil als gesondertes, auf den Rahmen und/oder die Sichtscheibe bzw. die Sichtscheiben aufsetzbares Teil ausgebildet ist, oder alternativ, daß das Nasenauflageteil einstückig mit dem Rahmen ausgebildet ist. In beiden Fällen ist es möglich, das Polsterteil unmittelbar anzuspritzen.

Sowohl bei der Herstellung von Polsterteilen im Nasenbereich als auch am Bügelende ist es möglich, das Polsterteil durch gemeinsames Spritzen mit dem Kunststoff des Basismaterials zu realisieren, was insbesondere im Bereich des Bügelendes mit hervorragenden Ergebnissen realisiert werden konnte.

Nachfolgend wird die Erfindung anhand bevorzugter Ausführungsbeispiele in Verbindung mit der Zeichnung näher beschrieben. Dabei zeigen:
- Fig. 1: eine Seitenansicht eines erfindungsgemäß ausgestalteten Brillenbügels,
- Fig. 2: einen Schnitt längs der Linie II-II in Fig. 1,
- Fig. 3: eine Aufsicht auf den Brillenbügel nach Fig. 1,
- Fig. 4: eine perspektivische Ansicht des Nasenbereiches einer erfindungsgemäßen Brille und
- Fig. 5: einen Schnitt längs der Linie IV-IV in Fig. 4.

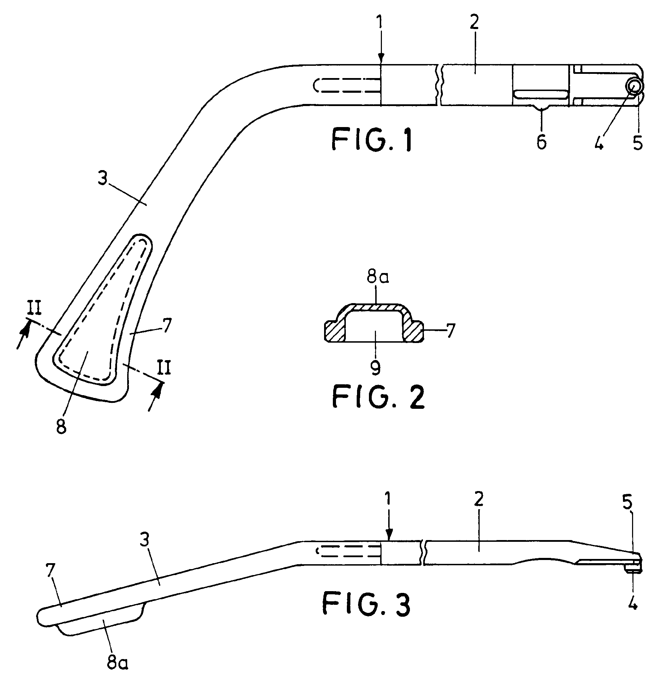

In Fig. 1 bis 3 ist ein Bügel 1 für eine Brille, z.B. einer Arbeitsschutzbrille, dargestellt, der einen im wesentlichen geraden, vorderen Scharnierabschnitt 2 und einen gegenüber dem Scharnierabschnitt 2 abgebogenen Ohrbügelabschnitt 3 aufweist.

Der Scharnierabschnitt 2 weist einen Scharnierlagerzapfen 4 am freien Vorderende 5 und einen Rastvorsprung 6 zur Bewerkstelligung einer Längenverstellbarkeit auf.

Der Ohrbügelabschnitt 3 weist ein verbreitertes Ende auf, an welchem ein Polsterteil 8 ausgebildet ist.

Wie insbesondere in Fig. 2 und 3 erkennbar ist, wird das Polsterteil 8 dadurch gebildet, daß das verbreiterte Ende 7 des Bügels 1 eine Ausbauchung 8a aufweist, welche membranartig dünnwandig ist und eine Ausnehmung 9 in dem Ende 7 des Bügels 1 überspannt und gegenüber dem Bügelende 7 nach innen vorgewölbt ist, so daß diese Auswölbung 8a weich und elastisch ist.

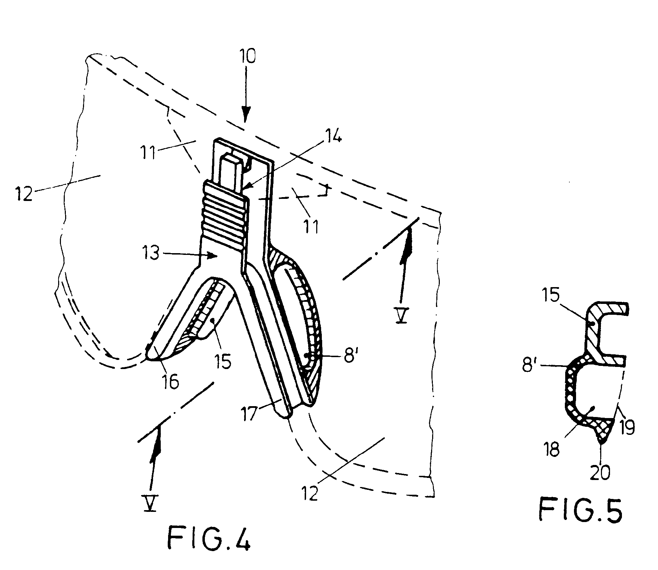

In Fig. 4 und 5 ist die Realisierung des Polsterteils 8′ im Nasenauflagebereich dargestellt. Die in Fig. 4 wiedergegebene Brille 10 umfaßt, wie gestrichelt angedeutet, Halteteile 11 für die Sichtscheiben 12. Im Nasenbereich ist ein gesondertes Nasenauflageteil 13 angeordnet, welches über eine Rastanordnung 14 in das darüberliegende Rahmen- und Halteteil 11 einrastbar ist.

Das Nasenauflageteil 13 umfaßt einen tragenden Grundkörper 15, der an seiner Unterseite V-förmig ausgebildet ist, wobei die V-Schenkel 16,17 jeweils durch außen offene U-Profile gebildet sind. An der Rück- bzw. Innenseite jedes derartigen Profils schließt sich ein im Zwei-Komponenten-Spritzverfahren unmittelbar angespritztes Polsterteil 8′ aus einem relativ weichen Kunststoff an.

Jedes Polsterteil 8′ weist eine blasenartig aufgewölbte Konfiguraton auf, wobei ein Hohlraum 18 ausgebildet wird, der zur Nasenaußenseite 16 hin offen ist. Der freie Außenrand 20 des so gebildeten Polsterteils 8′ ist wulstartig verstärkt ausgebildet.

## Patentansprüche

1. Brille mit einem Rahmen und Bügeln aus einem relativ harten Kunststoff bzw. Metall umfassend wenigstens ein Polsterteil aus einem relativ weichen Kunststoff an den Bügelenden und/oder Nasenauflageteilen, **dadurch** **gekennzeichnet, daß** das Polsterteil (8,8′) als aus Kunststoff angespritzte, relativ dünnwandige Aufwölbung ausgebildet ist.

2. Brille nach Anspruch 1, **dadurch gekennzeichnet**, **daß** an einem tragenden Grundkörper (15) eines Nasenauflageteils (13) bzw. des Ohrabschnitts (3) des Bügels (1) aus relativ hartem Kunststoff ein Polsterteil (8,8′) aus relativ weichem Kunststoff einstückig angespritzt ist.

3. Brille nach Anspruch 2, **dadurch gekennzeichnet**, **daß** das Polsterteil (8,8′) eine blasenartig aufgewölbte Konfiguration derart aufweist, daß die konvex vorgewölbte Außenseite dem Nasenrücken bzw. Kopf des Trägers federnd anliegt.

4. Brille nach Anspruch 3, **dadurch gekennzeichnet**, **daß** das blasenartig aufgewölbte Polsterteil (8,8′) einen etwa ovalen Grundflächenbereich umschließt, der in dem der Auflageseite abgewandten Bereich offen ist.

5. Brille nach Anspruch 2, **dadurch gekennzeichnet**, **daß** das Polsterteil (8,8′) einen verstärkten, wulstartigen freien Außenrand (20) aufweist.

6. Brille nach Anspruch 1, **dadurch gekennzeichnet**, **daß** das Nasenauflageteil (13) als gesondertes, auf den Rahmen (11) und/oder die Sichtscheibe bzw. die Sichtscheiben (12) aufsetzbares Teil ausgebildet ist.

7. Brille nach Anspruch 1, **dadurch gekennzeichnet**, **daß** das Nasenauflageteil (13) einstückig mit dem Rahmen (11) ausgebildet ist.

8. Brille nach Anspruch 1, **dadurch gekennzeichnet, daß** das Polsterteil (8,8′) mit dem tragenden Grundkörper (15) bzw. (3) in einem Spritzvorgang gespritzt ist.

## Claims

1. A pair of goggles with a frame and temples made of relatively hard plastic material or of metal comprising at least one pad element made of relatively soft plastic material at the ends of the temples and/or nose contact elements, characterized in that the pad element (8, 8′) is formed as a relatively thin-walled convexity which is moulded on of plastic material.

2. A pair of goggles according to claim 1, characterized in that at a supporting basic body (15) of a nose contact element (13) or of the ear section (3) of the temple (1) made of relatively hard plastic material a pad element (8, 8′) made of relatively soft plastic material is moulded on in one piece.

3. A pair of goggles according to claim 2, characterized in that the pad element (8, 8′) has a bubbles-like convexed configuration in such a manner that the convexity resiliently abuts on the nose bridge or on the head of the person wearing the goggles.

4. A pair of goggles according to claim 3, characterized in that the bubbles-like convexed pad element (8, 8′) embraces an approximately oval basic surface region, which is open in the region facing away from the side of contact.

5. A pair of goggles according to claim 2, characterized in that the pad element (8, 8′) has a reinforced, convexed free outer edge (20).

6. A pair of goggles according to claim 1, characterized in that the nose contact element (13) is formed as a separate element, which can be placed onto the frame (11) and/or the sight piece or sight pieces (12).

7. A pair of goggles according to claim 1, characterized in that the nose contact element (13) is formed in one piece with the frame (11).

8. A pair of goggles according to claim 1, characterized in that the pad element (8, 8′) with the supporting basic body (15) or (3) is moulded in one injection moulding process.

## Revendications

1. Lunettes comprenant une monture et des branches en matière plastique relativement dure ou en métal, munie d'au moins une partie rembourrée en matière plastique relativement tendre aux extrémités des branches et/ou sur les parties d'appui nasal, caractérisées en ce que la partie rembourrée (8, 8′) est conçue sous la forme d'un bombement à paroi relativement mince obtenu par injection de matière plastique.

2. Lunettes selon la revendication 1, caractérisées en ce qu'une partie rembourrée (8, 8′) en matière plastique relativement tendre est injectée d'un seul tenant avec le corps de base porteur (15) de la partie d'appui nasal (13) ou du segment de contour d'oreille (3) de la branche (1) en matière plastique relativement dure.

3. Lunettes selon la revendication 2, caractérisées en ce que la partie rembourrée (8, 8′) présente une configuration bombée en forme de bulle, de sorte que le côté extérieur à bombement convexe est appliqué de manière élastique contre l'arête nasale ou la tête du porteur.

4. Lunettes selon la revendication 3, caractérisées en ce que la partie rembourrée bombée en forme de bulle (8, 8′) comprend une zone de surface de base sensiblement ovale qui est ouverte dans la zone opposée au côté d'appui.

5. Lunettes selon la revendication 2, caractérisées en ce que la partie rembourrée (8, 8′) comporte un bord extérieur libre renforcé en forme de bourrelet (20).

6. Lunettes selon la revendication 1, caractérisées en ce que la partie d'appui nasal (13) est conçue sous la forme d'une pièce distincte pouvant être montée sur la monture (11) et/ou sur le ou les verres (12).

7. Lunettes selon la revendication 1, caractérisées en ce que la partie d'appui nasal (13) est conçue d'un seul tenant avec la monture (11).

8. Lunettes selon la revendication 1, caractérisées en ce que la partie rembourrée (8, 8′) est réalisée avec le corps de base porteur (15) ou (3) en une seule opération de moulage par injection.
